# EUROPÄISCHE PATENTSCHRIFT

(11) **EP 1 267 731 B1**
(45) Veröffentlichungstag und Bekanntmachung des Hinweises auf die Patenterteilung: **27.06.2007**
(21) Anmeldenummer: 01915384.0
(22) Anmeldetag: 20.03.2001
(51) Int. Cl.: A61B 17/16

(54) **VORRICHTUNG ZUM BEARBEITEN VON MENSCHLICHEM UND/ODER TIERISCHEM GEWEBE**
DEVICE FOR TREATING HUMAN AND/OR ANIMAL TISSUE
DISPOSITIF PERMETTANT DE TRAITER DES TISSUS HUMAINS ET/OU ANIMAUX

(30) Priorität: 05.04.2000 DE 20006247 U
(43) Veröffentlichungstag der Anmeldung: 02.01.2003
(73) Patentinhaber: Joist, Alexander, 49808 Lingen (DE)
(72) Erfinder: Joist, Alexander, 49808 Lingen (DE)
(74) Vertreter: von Kirschbaum, Alexander
(86) Internationale Anmeldenummer: PCT/EP2001/003168
(87) Internationale Veröffentlichungsnummer: WO 2001/076491

(56) Entgegenhaltungen:
- EP-A- 0 861 635
- WO-A-97/03611
- US-A- 4 706 659
- US-A- 5 624 393
- US-A- 5 694 951
- US-A- 5 849 023
- US-A- 5 913 859
- US-A- 5 947 972

## Beschreibung

Die Erfindung betrifft eine Vorrichtung zum Bearbeiten von menschlichem und/oder tierischem Gewebe, insbesondere ein Arthoskop, ein Endoskop oder einen Kernbohrer, insbesondere einen Markraumbohrer für Röhrenknochen, beispielsweise Oberschenkelknochen.

Mit Hilfe der Arthoskopie wird in menschliche oder tierische Gelenkhöhlen vorgedrungen, um beispielsweise die Oberfläche eines Knochens zu bearbeiten. Bei der Bearbeitung handelt es sich beispielsweise um das Glätten der Oberfläche. Mit Hilfe der Arthoskopie erfolgen ferner auch Gewebeentnahmen, wie das Entnehmen von Knochenteilen aus Gelenken durch Fräsen, Schaben u.dgl. Bei der Endoskopie erfolgt eine ähnliche Bearbeitung von menschlichem Gewebe, wobei die Endoskopie jedoch nicht in Gelenkhöhlen, sondern in beliebigen anderen Bereichen des menschlichen oder tierischen Körpers eingesetzt wird.

Bei Knochenbrüchen von langen Röhrenknochen, wie Oberschenkelknochen, werden die beiden Knochenteile zunächst ausgerichtet und anschließend in Längsrichtung mit einer Bohrung versehen. Anschließend wird in die Bohrung ein Nagel zur Fixierung der beiden Knochenteile eingeführt.

Die Bohrung erfolgt, indem zunächst ein Führungsspieß in das Knochenmark der beiden Knochenteile gesteckt wird. Bei dem Führungsspieß handelt es sich um einen Draht, mit einem Durchmesser von 2 bis 3 mm. Der Führungsspieß dient zum Führen eines Bohrkopfes. Der Bohrkopf ist mit einer flexiblen Welle verbunden, die mit einem Bohrantrieb, beispielsweise eine pneumatische Bohrmaschine angetrieben wird.

Beim Herstellen der Bohrung entsteht am Bohrkopf ein hoher Druck. Durch den Druck werden Knochen- und Fettpartikel in Venen gepreßt. Hierbei kann es vorkommen, dass die Partikel über die Blutbahn in die Lunge transportiert werden und zu einer Lungenembolie führen. Insbesondere wenn zusätzlich zu dem Knochenbruch auch die Lunge verletzt ist, kann eine Fixierung der beiden Knochenteile durch die vorstehend beschriebene Nagelung nicht erfolgen, da das Risiko einer Lungenembolie zu groß ist. Der Knochen kann daher nur von außen fixiert werden. Dies hat zur Folge, dass der Bruch häufig schlecht verheilt, eine anatomisch korrekte Einrichtung des Bruches schwierig ist, häufig Infektionen des Weichteilmantels erfolgen und ggf. in einer zweiten Operation die definitive Bruchversorgung vorgenommen werden muss.

Zur Verringerung des Drucks am Bohrkopf ist aus CH 687 228 A5 ein spezieller Bohrkopf für die Markraumbohrung bekannt. Der Bohrkopf weist Öffnungen auf, durch die das Bohrmaterial, d.h. in erster Linie das Knochenmark, nach hinten transportiert werden kann. Da sich in dem Bohrloch jedoch eine flexible Welle befindet, ist nur wenig Raum zur Aufnahme des Bohrmaterials. Daher ist die Menge des Bohrmaterials größer als der Raum hinter dem Bohrer, so dass das Bohrmaterial weiterhin zusammengepreßt wird. Somit baut sich im Bereich des Bohrkopfes Druck auf. Dies kann zur Ausschwemmung von Bohrmaterial über die Blutbahn in die Lunge und damit zu Lungenembolie führen.

Die Probleme des erhöhten Druckaufbaus sind auch beim Einsatz von Endoskopen und Arthoskopen nachteilig und können jeweils zu den vorstehend genannten Problem führen.

Aufgabe der Erfindung ist es, eine Vorrichtung zur Bearbeitung von menschlichem und/oder tierischem Gewebe, insbesondere einen Kernbohrer, zu schaffen, der insbesondere zur Markraumbohrung für Röhrenknochen geeignet ist, durch den ein Druckaufbau am Bearbeitungskopf vermieden ist.

Die Lösung dieser Aufgabe erfolgt durch die Merkmale des Patentanspruchs 1.

Handelt es sich bei der erfindungsgemäßen Vorrichtung um einen Kernbohrer, so weist dieser eine Spüleinheit auf, durch die das Bohrmaterial vom Bereich des Bearbeitungskopfes abtransportiert wird. Bei einem Kernbohrer ist als Bearbeitungskopf ein Bohrkopf vorgesehen. Durch eine kontinuierliche Spülung wird das abgetragene Material, d.h. das Bohrmaterial, beispielsweise aus dem Markraum des Knochens herausgespült, so dass innerhalb des Knochens und insbesondere am Bohrkopf kein Druck entstehen kann. Aufgrund des erfindungsgemäßen drucklosen Kernbohrers ist ein Ausschwemmen von Bohrmaterial in die Lunge und damit die Gefahr von Lungenembolien vermieden.

Erfindungsgemäß weist der Kernbohrer eine flexible Welle mit Innenkanal auf. Ferner weist der mit der flexiblen Welle verbundene Bohrkopf Öffnungen auf, die mit dem Innenkanal verbunden sind. Erfindungsgemäß ist eine Spüleinheit vorgesehen, wobei eine Anlagefläche der Spüleinheit zum im wesentlichen abdichtenden Aufsetzen auf einen Gegenstand vorgesehen ist. Wird der Kernraumbohrer zur Markraumaufbohrung eingesetzt, handelt es sich bei dem Gegenstand um einen Röhrenknochen. Ferner ist erfindungsgemäß der Durchmesser des Bearbeitungskopfes, wie des Bohrkopfes, größer als der Außendurchmesser der flexiblen Welle, so dass durch die äußere Mantelfläche der flexiblen Welle und ein in dem Gegenstand, beispielsweise dem Röhrenknochen, erzeugtes Bohrloch ein Spülkanal gebildet ist.

Durch die Spüleinheit wird beim Bohren von Röhrenknochen von der Spüleinheit ein Spülmedium in den Spülkanal eingeleitet. Das Spülmedium fließt somit entlang der äußeren Mantelfläche der flexiblen Welle in Richtung des Bohrkopfes. Da die Spüleinheit eine Anlagefläche aufweist, die im wesentlichen gegenüber einer Knochenoberfläche abdichtet, kann auf einfache Weise das Spülmedium in den Spülkanal geleitet werden. Am Bohrkopf gelangt das Spülmedium durch die in dem Bohrkopf vorgesehenen Öffnungen in das Innere des Bohrers. Hierbei wird von dem Spülmedium das Bohrmaterial aufgenommen und durch die im Bohrkopf vorgesehenen Öffnungen nach innen transportiert. Da die Öffnungen mit dem Innenkanal der flexiblen Welle verbunden sind, wird das Spülmedium in das Innere der flexiblen Welle transportiert. In dem Innenkanal der flexiblen Welle wird das Spülmedium zusammen mit dem Bohrmaterial in Richtung der Spüleinheit aus dem Knochen heraustransportiert.

Der Transport des Spülmediums kann ebenso in umgekehrter Richtung erfolgen, so dass das Spülmedium durch den Innenkanal der flexiblen Welle zum Bohrkopf gelangt, aus dessen Öffnungen austritt und durch den Spülkanal aus dem Knochen herausgeleitet wird. Die Abführung des Spülmediums durch den Innenkanal der flexiblen Welle hat jedoch den Vorteil, dass das Bohrmaterial nicht an der Innenwand der Bohrung entlanggeführt wird. Dies könnte zu Verstopfungen des Bohrlochs und damit ggf. zur Verletzung an der Innenwand der Bohrung führen.

Das Vorsehen einer Spüleinheit hat ferner den Vorteil, dass am Bohrkopf keine Hitzeentwicklung auftritt, die zu Gewebeveränderungen führen kann.

Vorzugsweise weist die Spüleinheit eine Saugvorrichtung auf, um an dem Bohrkopf Unterdruck zu erzeugen. Durch die Erzeugung von Unterdruck ist sichergestellt, dass sämtliches Bohrmaterial sofort abgeführt wird. Hierdurch ist vermieden, dass am Bohrkopf Druck auftritt, durch den Bohrmaterial in die Blutbahn des Patienten gelangen kann. Vorzugsweise ist die Saugvorrichtung mit dem Innenkanal der flexiblen Welle verbunden, so dass die Absaugung des Spülmediums zusammen mit dem Bohrmaterial durch den Innenkanal erfolgt. Dies hat den Vorteil, dass die Abdichtung zwischen der Anlagefläche der Spüleinheit und dem Gegenstand nicht besonders gut abdichtend ausgeführt sein muss, da bei dieser Saugrichtung an der Anlagefläche keine hohen Drücke auftreten.

Vorzugsweise weist die flexible Welle mehrere gelenkig miteinander verbundene Wellenglieder auf. Durch das Vorsehen mehrerer Wellenglieder kann ein gleichmäßiges Drehmoment übertragen werden. Gegenüber bekannten flexiblen Wellen aus spiralförmig gewickeltem Draht weist die flexible Welle aus Wellengliedern den Vorteil auf, dass beispielsweise ein Verhaken und anschließendes schlagartiges freies Weiterdrehen des Bohrkopfs durch Entspannen der flexiblen Welle nicht auftritt. Bei flexiblen Wellen aus spiralförmig gewickeltem Draht kann durch ein Verdrillen des Drahtes ein entsprechendes Drehmoment aufgebaut werden. Bei plötzlichem Freiwerden des Bohrkopfes entspannt sich die flexible aus verdrilltem Draht. Hierdurch werden die übertragenden Drehmomente ungleichmäßig.

In der mehrere Wellenglieder aufweisenden flexiblen Welle ist vorzugsweise ein Schlauch vorgesehen. Durch den Schlauch ist der Innenkanal der flexiblen Welle gegenüber dem Spülkanal abgedichtet, da ansonsten zwischen den einzelnen Wellengliedern Bohrmaterial in den Spülkanal gelangen könnte. Ferner könnte durch das Bohrmaterial die Flexibilität der Welle beeinträchtigt werden, wenn sich zwischen den einzelnen Wellengliedern Bohrmaterial sammelt. Bei umgekehrter Transportrichtung des Spülmediums, d.h. falls Bohrmaterial durch den Spülkanal abgeführt wird, sollte die flexible Welle von einem Schlauch umgeben sein, um das Ablagern von Bohrmaterial zwischen den Wellengliedern zu vermeiden.

Die Spüleinheit, die außen auf den Gegenstand bzw. auf den Knochen aufgesetzt wird, weist vorzugsweise einen Spülkopf auf. In dem Spülkopf ist die flexible Welle drehbar gehalten. Das Spülmedium, das durch den Innenkanal der flexiblen Welle nach außen transportiert wird, gelangt aus dem Innenkanal in den Spülkopf und wird durch diesen abgeführt. Hierzu weist die flexible Welle im Bereich des Spülkopfes Öffnungen und der Spülkopf eine Ringnut auf, so dass trotz Drehung der flexiblen Welle das Spülmedium zusammen mit dem Bohrmaterial aus dem Innenkanal in die Ringnut und aus dieser nach außen abgeführt werden kann.

Vorzugsweise ist der Spülkopf in einem Führungselement der Spüleinheit verschiebbar gelagert. Hierdurch kann die Anlagefläche der Spüleinheit fest auf den Gegenstand bzw. dem Knochen gehalten werden und dient gleichzeitig als Führung der flexiblen Welle sowie des Bohrkopfes. Die flexible Welle wird so zusammen mit dem Bohrkopf und dem Spülkopf in dem Führungselement zur Herstellung der Bohrung in dem Gegenstand bzw. dem Knochen geführt.

Die flexible Welle weist vorzugsweise mehrere Wellenglieder auf. Jedes Wellenglied weist mindestens einen Ansatz und eine Ausnehmung auf, der in eine Ausnehmung bzw. einen Ansatz eines benachbarten Wellenglieds eingreift, so dass Ansätze und Ausnehmungen ineinandergreifen. Die Abmessungen der Ansätze und Ausnehmungen sind so aufeinander abgestimmt, dass ein um den Wellenumfang umlaufender Spalt ausgebildet ist. Die ineinandergreifenden Ansätze und Ausnehmungen benachbarter Wellenglieder sind ferner so ausgebildet, dass sie sich einander in Längsrichtung hintergreifen. Dadurch sind die Wellenglieder in Längsrichtung an der flexiblen Welle unlösbar miteinander verbunden. Aufgrund des umlaufenden Spalts entsteht ein gegenseitiges Spiel zwischen den einzelnen Wellengliedern.

In Abhängigkeit der Größe des Spiels sowie der Länge der einzelnen Wellenglieder ist die Welle mehr oder weniger flexibel. Durch das Drehen eines mit einem Antrieb verbundenen Wellengliedes wird der Spalt zwischen den Wellengliedern teilweise geschlossen, so dass sich die einander gegenüberliegende Spaltflächen teilweise berühren. Über diese Berührungsbereiche wird von einer auf das nächste Wellenglied Kraft übertragen. Auf diese Weise wird von der flexiblen Welle ein Drehmoment übertragen. Zur Übertragung großer Drehmomente kann die Form der Ansätze und Ausnehmungen so ausgebildet sein, dass beim Drehen der Welle möglichst große Berührungsflächen vorhanden sind.

Die einzelnen Wellenglieder sind vorzugsweise in sich steif. Sie bestehen vorzugsweise aus hartem Kunststoff oder Metall und insbesondere aus Titan. Da der Ansatz eines Wellenglieds in die Ausnehmung eines benachbarten Wellenglieds eingreift, hintergreift der Ansatz erfindungsgemäß die Ausnehmung. Der Ansatz weist somit einen halsförmigen und einen kopfförmigen Teil auf. Ebenso weist die Ausnehmung einen halsförmigen und einen kopfförmigen Teil auf. Hierdurch sind die Wellenglieder in Längsrichtung unlösbar miteinander verbunden. Auch in der Ausgestaltung der Ansätze und der Ausnehmungen können durch die erfindungsgemäße flexible Welle auch Zug- und Druckkräfte übertragen werden. Die Bereiche der Spaltflächen, an denen die wellenglieder einander bei der Übertragung von Zug- oder Druckkräften berühren, können vorzugsweise wiederum so ausgebildet sein, dass eine Übertragungsfläche ausgebildet ist, um die Flächenpressung zu verringern.

Vorzugsweise weist jedes Wellenglied am Wellenumfang mindestens zwei Ansätze und zwei Ausnehmungen auf. Hierdurch wird die Fläche für die Kraftübertragung zwischen zwei Wellengliedern erhöht. Vorzugsweise sind die Ansätze gleich groß und regelmäßig am Umfang der Welle angeordnet. Hierdurch ist gewährleistet, dass die flexible Welle in jede Biegerichtung eine annähernd gleiche Flexibilität aufweist.

Vorzugsweise ist das Verhältnis von Spaltbreite zu Wellendurchmesser bei runden Wellengliedern zwischen 1 : 100 und 1 : 10. Dies bedeutet bei einem Wellendurchmesser von 10 mm, dass die Spaltbreite zwischen 0,1 mm und 1 mm beträgt. Durch die Wahl der Spaltbreite kann die maximal mögliche Krümmung der flexiblen Welle beeinflußt werden. Um die Zuverlässigkeit der Übertragung von Drehmomenten sowie von Kräften zu erhöhen, ist es vorteilhaft, möglichst geringe Spaltbreiten vorzusehen, d.h. bei einem Wellendurchmesser von 10 mm eine Spaltbreite von lediglich 0,5 bis 0,1 mm vorzusehen. Zur Erhöhung der Flexibilität sollte nicht die Spaltbreite vergrößert werden, sondern die Anzahl der Wellenglieder erhöht werden, in dem die Länge der einzelnen Wellenglieder verringert wird.

Im medizinischen Bereich ist der erfindungsgemäße Kernbohrer nicht nur als Markraumbohrer sondern beispielsweise auch zum Lösen und Herausspülen von Gefäßverkalkungen sowie zum Entfernen von Nieren-, Harnleiter- und Gallensteinen geeignet. Hierzu kann am Bohrkopf zusätzlich eine Greif- oder Einfangvorrichtung vorgesehen sein. Die zertrümmerten Steine werden erfindungsgemäß durch die Spülung abtransportiert.

Vorstehend beschriebener Kernbohrer ist nicht nur für medizinische Zwecke, sondern generell zur Herstellung von Kernbohrungen geeignet, bei denen die Erzeugung von Druck an dem Bohrkopf vermieden werden soll. Dies ist beispielsweise der Fall, wenn das das Bohrloch umgebende Material durch den beim Bohren entstehenden Druck beschädigt werden könnte. Der erfindungsgemäße Bohrer ist daher insbesondere zum Bohren von weichen Kunststoffen und Naturmaterialien geeignet. Da bei dem erfindungsgemäßen Kernbohrer kein Druck am Bohrkopf aufgebaut wird, ist der Bohrer für spröde Materialien besonders gut geeignet, da diese durch den Druck beschädigt werden können. Beispielsweise können in dem Material Haarrisse auftreten.

Der erfindungsgemäße Kernbohrer kann sowohl im großtechnischen als auch in der Mikro- und Nanotechnologie eingesetzt werden.

Nach Fertigstellung der Bohrung kann durch den Innenkanal der flexiblen Welle Material in den gebohrten Gegenstand eingebracht werden. Somit kann direkt beim Herausziehen des Bohrers das Bohrloch mit Füllmaterial, z.B. zur Versteifung des Gegenstandes gefüllt werden.

Bei einer bevorzugten erfindungsgemäßen Weiterbildung kann die flexible Welle selbst als Nagel zur Verbindung zweier Knochenstücke dienen. Hierzu ist die flexible welle mit einer Spannvorrichtung zur Versteifung der Welle verbunden oder verbindbar. Durch die Spannvorrichtung werden die zwischen den einzelnen Wellengliedern bestehenden Spalten geschlossen. Durch die Spannvorrichtung werden die einzelnen Wellenglieder somit zusammengezogen. Da der Spalt derart ausgebildet ist, dass zwischen den Wellengliedern Tangentialkräfte übertragen werden können, entsteht durch das Zusammenziehen der Wellenglieder ein unflexibles Rohr. Dies kann direkt als Nagel in einem Knochen dienen. Das Zusammenziehen der einzelnen Wellenglieder kann beispielsweise durch einen geeigneten Seilzug oder durch Stauchen der flexiblen Welle geschehen.

Vorzugsweise weist die flexible Welle ferner eine Verankerungsvorrichtung zum Verankern der Welle in dem Knochen auf. Eine derartige Verankerungsvorrichtung ist vorzugsweise derart ausgebildet, dass beim Spannen der flexiblen Welle Verankerungskeile o.dgl. nach außen klappen und ein Verankern der zu einem Nagel versteiften flexiblen Welle in dem Knochen bewirken. Selbstverständlich kann das Verankern auch vor oder nach dem Versteifen der Welle erfolgen. Es ist ferner auch möglich, gesondert Verankerungselemente in den Innenkanal der flexiblen Welle einzubringen und diese durch entsprechende in der Welle vorgesehene Öffnungen einzustecken, so dass hierdurch eine Verankerung der Welle in dem Knochen erfolgt.

Die Schneiden des Bohrkopfes sind vorzugsweise derart ausgebildet, dass der Bohrkopf, sobald er von einem weicheren Kernmaterial auf eine härtere Ummantelung trifft, in das Kernmaterial zurückgelenkt wird. Dadurch ist vermieden, dass der Bohrer aus dem zu bohrenden Gegenstand, wie dem Knochen, seitlich austritt.

Die vorstehend am Beispiel des Kernbohrers beschriebene erfindungsgemäße Vorrichtung zur Bearbeitung von menschlichem und/oder tierischem Gewebe ist ebenso als Arthoskop oder Endoskop einsetzbar. Das erfindungsgemäße Arthoskop oder Endoskop weist hierbei ebenfalls eine flexible Welle mit Innenkanal auf. Die flexible Welle ist mit einem Bearbeitungskopf, mit dem Gewebe abgetragen werden kann, indem das Gewebe beispielsweise geglättet, gefräst, geschabt o.dgl. wird, verbunden. Der Bearbeitungskopf weist Öffnungen auf, die mit dem Innenkanal verbunden sind. Ferner weist das erfindungsgemäße Arthoskop oder Endoskop eine Spüleinheit auf. Entsprechend dem vorstehend beschriebenen Kernbohrer ist der Durchmesser des Bearbeitungskopfes, d.h. die maximalen Außenabmessungen des Bearbeitungskopfes, bei dem es sich nicht um ein rotierendes Werkzeug handeln muss, größer als der Außendurchmesser der flexiblen Welle. Hierdurch entsteht zur Durchleitung eines Spülmediums ein durch die äußere Mantelfläche der flexiblen Welle und ein in dem Gegenstand, d.h. dem menschlichen Gewebe, erzeugtes Loch ein Spülkanal.

Bei dem erfindungsgemäßen Arthoskop kann ein zusätzlicher Arbeitskanal vorgesehen sein. Durch diesen kann beispielsweise eine Optik eingeführt werden, um den Bearbeitungsprozeß zu beobachten. Ferner kann durch den Arbeitskanal Spülflüssigkeit zugeführt werden, die sodann über den Bearbeitungskopf und den zwischen der flexiblen Welle und der Innenwand des Bohrlochs ausgebildeten Spülkanal abgesaugt werden kann.

Ein erfindungsgemäßes Arthoskop oder Endoskop kann entsprechend dem vorstehend beschriebenen Kernbohrer bevorzugt weitergebildet sein.

Nachfolgend wird die Erfindung anhand einer bevorzugten Ausführungsform unter Bezugnahme auf die anliegenden Zeichnungen näher erläutert.

Es zeigen:

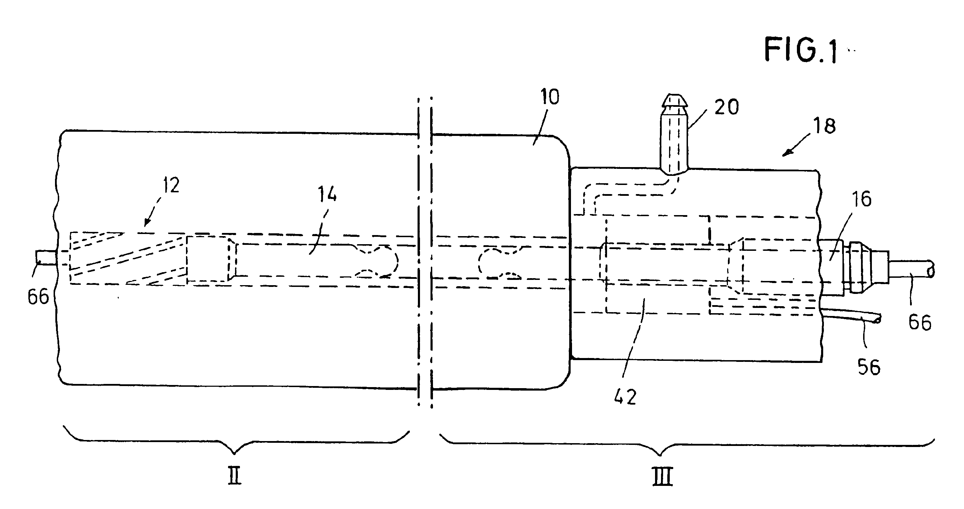

- Fig. 1: eine schematische Seitenansicht des Kernbohrers,
- Fig. 2: eine schematische Schnittansicht des Bereichs II in Fig. 1, und

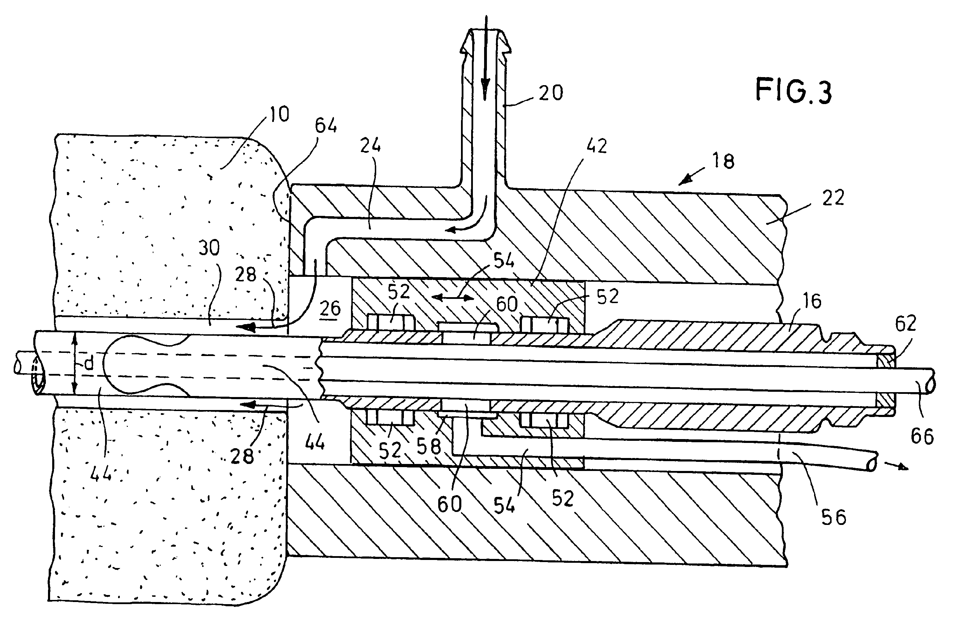

- Fig. 3: eine schematische Schnittansicht des Bereichs III in Fig. 1.

Der Kernbohrer kann erfindungsgemäß insbesondere zum Erzeugen einer Kernbohrung in einem Röhrenknochen 10, wie beispielsweise einem Oberschenkelknochen verwendet werden. Hierzu weist der Kernbohrer einen Bohrkopf 12 auf, der mit einer flexiblen Welle 14 verbunden ist. Die flexible Welle 14 ist mittels eines Schafts 16 über ein Bohrfutter o.dgl. mit einer Bohrmaschine, beispielsweise einer pneumatischen Bohrmaschine verbunden. Der Kernbohrer weist ferner eine Spüleinheit 18 zum Abtransportieren des im Bereich des Bohrkopfes 12 entstehenden Bohrmaterials mit Hilfe eines Spülmediums auf. Als Spülmedium wird im medizinischen Bereich vorzugsweise eine Kochsalzlösung eingesetzt. In anderen technischen Bereichen können auch Gase oder andere flüssige Medien als Spülmedium verwendet werden.

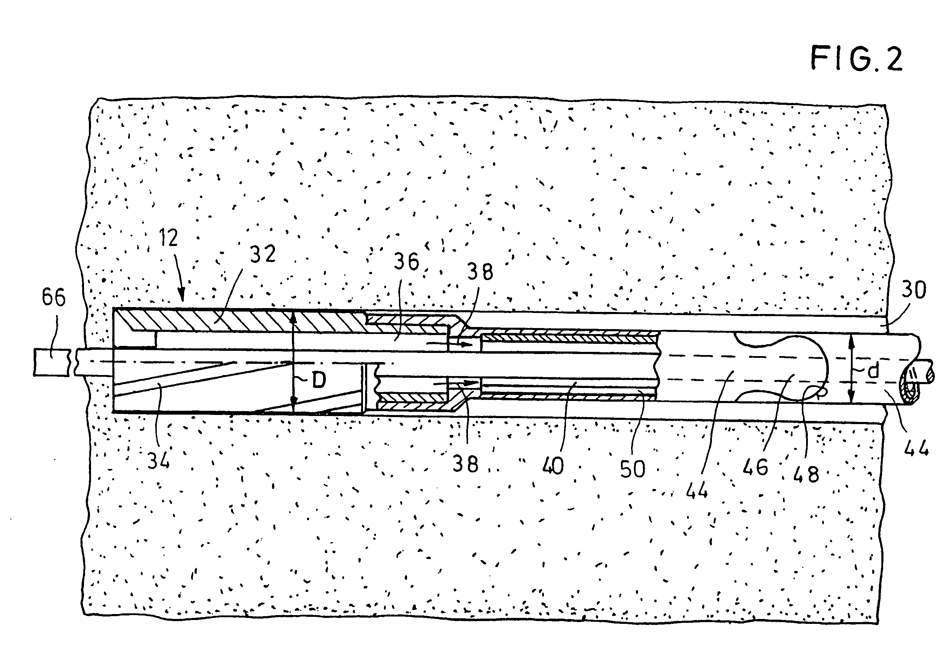

Die Spüleinheit 18 weist einen Anschluß 20 auf, der mittels eines Schlauchs mit einem Vorratsbehälter, in dem Spülmedium vorhanden ist verbunden ist. Das Spülmedium wird durch ein in einem Führungselement 22 des Spülkopfes 18 vorgesehenen Kanal 24 geleitet. Aus dem Kanal 24 wird das Spülmedium in einen zylindrischen Hohlraum 26, der sich innerhalb des Führungselements 22 befindet, geleitet. Aus diesem gelangt das Spülmedium in Richtung der Pfeile 28 in einen Spülkanal 30. Der Spülkanal 30 ist zwischen einem Außendurchmesser d, der flexiblen Welle 14 und dem Bohrungsdurchmesser D (Figur 2) ausgebildet. Der Bohrungsdurchmesser D entspricht dem Bohrdurchmesser D des Bohrkopfes 12. Das Spülmedium gelangt durch den Spülkanal 30 zum Bohrkopf 12 und wird entlang Schneiden 32 des Bohrkopfes 12 geleitet. An den Schneiden 32 des Bohrkopfes 12 nimmt das Spülmedium Bohrmaterial auf. Beim Bohren von Röhrenknochen handelt es sich hierbei in erster Linie um Knochenmark. Das Knochenmark wird von dem Spülmedium abtransportiert. Hierzu weist der Bohrkopf Öffnungen in Form von Längsschlitzen 34 auf, durch die das Spülmedium ins Innere des Bohrkopfes 12 gelangen.

Der Bohrkopf 12 ist im Inneren hohl, so dass das Spülmedium durch die Schlitze 34 in den hohlen Innenbereich 36 des Bohrkopfes 12 gelangt. Aus dem hohlen Innenbereich 36 des Bohrkopfes 12 gelangt das Spülmedium in Richtung der Pfeile 38 in einen in der flexiblen Welle ausgebildeten Innenkanal 40. Die flexible Welle 14 ist über ihre gesamte Länge hohl, so dass sich der Innenkanal 40 vom Bohrkopf 12 bis zur Spüleinheit 18 erstreckt. Das Spülmedium fließt zusammen mit dem Bohrmaterial durch den Innenkanal 40 zu einem in der Spüleinheit 18 vorgesehenen Spülkopf 42.

Die flexible Welle weist mehrere Wellenglieder 44 auf. Jedes Wellenglied weist mehrere Ansätze 46 auf, die in Ausnehmungen 48 des benachbarten Wellenglieds 44 eingreifen. Die Ansätze 46 und die Ausnehmungen 48 sind so ausgestaltet, dass sich die Ansätze 46 und die Ausnehmungen 48 in Längsrichtung der flexiblen Welle 14 hintergreifen. Dadurch sind die Wellenglieder 44 in Längsrichtung unlösbar miteinander verbunden. Um die Flexibilität der Welle 14 zu gewährleisten, ist zwischen den einzelnen Wellengliedern 44 ein sich über den gesamten Umfang der flexiblen Welle 14 erstreckender Spalt ausgebildet. Die Spaltbreite und die Länge der einzelnen Wellenglieder 44 definieren die Flexibilität der Welle 14. Die Ansätze 46 sowie die Ausnehmungen 48 weisen vorzugsweise die gleiche Größe auf und sind regelmäßig am Umfang der flexiblen Welle 14 angeordnet. Dadurch ist gewährleistet, dass die Flexibilität der Welle 14 in jede beliebige Auslenkrichtung gleich ist. Ferner weist der Spalt in Umfangsrichtung eine konstante Breite auf.

Da das Spülmedium aus dem Innenkanal 40 durch den zwischen den Wellengliedern 44 vorgesehenen Spalten in den Spülkanal 30 dringen könnte, und hierbei Bohrmaterial in die Spalte gelangen und damit die Flexibilität der Welle beeinflussen könnte, ist die flexible Welle 14 mit einem Schlauch 50 ausgekleidet. Der Schlauch 50 erstreckt sich vom Bohrkopf 12 bis zu dem Schaft 16.

Die flexible Welle 14, über die von der Bohrmaschine das Drehmoment zu dem Bohrkopf 12 übertragen wird, ist in dem Spülkopf 42 über Lager 52 drehbar gelagert. Ferner ist zum Durchführen der Bohrung der Spülkopf 42 in dem Führungselement 22 in Richtung des Pfeils 55, d.h. in Bohrrichtung verschiebbar gelagert. Der Spülkopf 42 ist hierzu vorzugsweise zylindrisch ausgebildet, so dass er in dem zylindrischen Hohlraum 26 des Führungselementes 22 verschiebbar ist. Um beim Bohren ein Mitdrehen des Spülkopfes 42 zu vermeiden, sind an der Innenwand des zylindrischen Hohlraums 26 sowie an der Mantelfläche des Spülkopfs 42 Führungselemente vorgesehen. Hierbei kann es sich um Nuten und entsprechende Ansätze handeln. Zwischen der äußeren Mantelfläche des Spülkopfs 42 und der Mantelfläche des zylindrischen Hohlraums 26 ist eine Dichtung vorgesehen, um zu verhindern, dass Spülmedium aus dem zylindrischen Hohlraum 26 an dem Spülkopf 42 vorbei nach außen dringt.

Das Spülmedium, das von dem Bohrkopf durch den Innenkanal 40 in der flexiblen Welle in Richtung des Spülkopfes 42 transportiert wird, tritt aus dem Spülkopf 42 durch einen Kanal 54 in einen Schlauch 56 ein, der mit einem Auffangbehälter verbunden ist. Hierzu weist der Spülkopf eine Ringnut 58 auf. Die flexible Welle 14 weist auf Höhe der Ringnut 58 mehrere Längsschlitze 60 auf, durch die das Spülmedium aus dem Innenkanal 40 in die Ringnut 58 fließt. Der Schlauch 56 ist vorzugsweise mit einer Saugvorrichtung verbunden, so dass am Bohrkopf 12 Unterdruck entsteht. Dadurch ist sichergestellt, dass beim Bohren des Knochens 10 im Bereich des Bohrkopfs keine Drücke auftreten, die zu einem Transport von Bohrmedium, d.h. beispielsweise von Knochenmark in Venen führen können. Somit ist die Gefahr einer Lungenembolie durch den Transport des Bohrmaterials in die Lunge vermieden.

Die flexible Welle kann an ihrer Innenseite, insbesondere an dem dem Bohrkopf 12 zugewandten Ende Ansätze aufweisen, durch die das Spülmedium in Rotation versetzt wird. Es handelt sich hierbei beispielsweise um schaufelartige Ansätze. Ferner kann eine Art archimedische Schraube vorgesehen sein. Ebenso kann die Innenseite des Schlauchs 56 mit einer schraubenlinienförmigen Nut oder einem schraubenlinienförmigen Ansatz versehen sein. Durch derartige Elemente wird das Spülmedium in Rotation versetzt und der Abtransport des Bohrmaterials verbessert.

Der Innenkanal 40 der flexiblen Welle 14 ist am oberen Bereich des Schaft 16 durch eine Dichtung 62 gegenüber der Bohrmaschine abgedichtet.

Das Führungselement 22 der Spüleinheit weist an der dem Knochen 10 zugewandten Seite eine Anlagefläche 64 auf, die an dem Knochen 10 anliegt. Durch die Anlagefläche wird die Spüleinheit 18 gegenüber dem Knochen 10 abgedichtet, so dass aus dem zylindrischen Hohlraum 26 kein oder nur geringe Mengen an Spülmedium entlang der Anlagefläche 64 nach außen in das den Knochen 10 umgebende Gewebe dringen kann. Das Austreten von Spülmedium entlang der Anlagefläche 64 wird zusätzlich dadurch vermieden, dass an dem Schlauch 56 eine Saugvorrichtung vorgesehen ist, so dass im Bereich des Hohlraums 26 keine erhöhten Drücke auftreten.

Um zu vermeiden, dass der Bohrkopf 12 des Kernbohrers aus dem Markraum des Knochens 10 durch die Außenwand des Knochens dringt, ist die Schneide des Bohrkopfes 12 so ausgebildet, dass der Bohrkopf 12, sobald er von innen gegen die Knochenwand, d.h. ein härteres Material trifft, wieder nach innen in den Markraum abgelenkt wird. Dadurch ist ein Perforieren des Knochens, d.h. ein Austreten des Bohrkopfes seitlich aus dem Knochen vermieden.

Dies kann auch durch Vorsehen eines Führungsdrahtes 66 erreicht werden. Der Führungsdraht 66 verläuft durch den Schaft 16, durch die hohle flexible Welle 14 und durch den Bohrkopf 12.

Zur Herstellung einer Kernbohrung wird beim Verwenden eines Führungsdrahtes 66 zuerst der Führungsdraht, der einen Durchmesser von etwa 2 bis 3 mm aufweist, durch den Knochen gesteckt. Beim anschließenden Bohren des Knochens 10 wird der Bohrkopf 12 automatisch von dem Führungsdraht 66 geführt, so dass der Bohrkopf 12 nicht seitlich aus dem Knochen 10 austreten kann. Zur Herstellung der Bohrung in dem Knochen 10 wird somit zuerst der Führungsdraht 66 eingeführt. Anschließend wird die Anlagefläche 64 der Spüleinheit 18 auf den Knochen aufgesetzt. Hierauf wird der Spülkopf 42 in den zylindrischen Hohlraum 26 des Führungselements 22 eingeführt, so dass der Bohrkopf 12 auf Höhe der Anlagefläche 64 den Knochen 10 berührt. Hierauf wird der Bohrkopf 12 durch die Bohrmaschine in Drehung versetzt und zusammen mit der flexiblen Welle 14 und dem Spülkopf 42 in Richtung des Pfeils 55 bewegt.

Die Spüleinheit 18 kann so ausgebildet sein, dass sie nicht als Führung für den Spülkopf 42 dient. Bei dieser Ausführungsform ist der Hohlraum 26 nicht gegenüber dem Spülkopf 42 abgedichtet, sondern gegenüber der äußeren Mantelfläche der flexiblen Welle 14. Dies hat den Vorteil, dass das Führungselement 22 nicht die gesamte Länge der Bohrung aufweisen muss.

## Patentansprüche

1. Vorrichtung zur Bearbeitung von menschlischem und/oder tierischem Gewebe, insbesondere Arthoskop, Endoskop oder Kernbohrer, insbesondere Markraumbohrer für Röhrenknochen, mit
einer flexiblen Welle (14) mit Innenkanal (40),
einem mit der flexiblen Welle (14) verbundenen Bearbeitungskopf (12) mit Öffnungen (34), die mit dem Innenkanal (40) verbunden sind, und
einer Spüleinheit (18) mit Anlagefläche (64) zum im wesentlichen abdichtenden Aufsetzen auf einen Gegenstand (10),
wobei ein Durchmesser (D) des Bearbeitungskopfes (12) größer als ein Außendurchmesser (d) der flexiblen Welle ist, so dass zur Durchleitung eines Spülmediums ein durch die äußere Mantelfläche der flexiblen Welle (14) und ein in dem Gegenstand (10) erzeugtes Bohrloch ein Spülkanal (30) gebildet ist.

2. Vorrichtung nach Anspruch 1, **dadurch gekennzeichnet, dass** die Spüleinheit (18) zur Erzeugung von Unterdruck an dem Bearbeitungskopf (12) mit einer Saugvorrichtung verbunden ist.

3. Vorrichtung nach Anspruch 2, **dadurch gekennzeichnet, dass** die Saugvorrichtung mit dem Innenkanal (40) der Welle (14) verbunden ist, so dass die Absaugung des Spülmediums zusammen mit dem abgetragenen Material durch den Innenkanal (40) erfolgt.

4. Vorrichtung nach einem der Ansprüche 1-3, **dadurch gekennzeichnet, dass** die flexible Welle (14) mehrere gelenkig miteinander verbundene Wellenglieder (44) aufweist, und in der flexiblen Welle (14) ein Schlauch (50) vorgesehen ist.

5. Vorrichtung nach einem der Ansprüche 1-4, **dadurch gekennzeichnet, dass** die Spüleinheit (18) einen Spülkopf (42) aufweist, in dem die flexible Welle (14) drehbar gehalten ist.

6. Vorrichtung nach Anspruch 5, **dadurch gekennzeichnet, dass** der Spülkopf (42) in einem Führungselement (22) der Spüleinheit (18) verschiebbar gelagert ist.

7. Vorrichtung nach einem der Ansprüche 1-6, **dadurch gekennzeichnet, dass** die flexible Welle (14) mehrere Wellenglieder (44) mit ineinandergreifenden Ansätzen (46) und Ausnehmungen (48) aufweist, die unter Bildung eines um den Wellenumfang umlaufenden Spaltes einander in Längsrichtung hintergreifen, so dass die Wellenglieder (44) in Längsrichtung unlösbar und mit gegenseitigem Spiel verbunden sind.

8. Vorrichtung nach Anspruch 7, **dadurch gekennzeichnet, dass** sämtliche Ansätze (46) die gleiche Größe aufweisen und regelmäßig am Umfang der Welle (14) angeordnet sind.

9. Vorrichtung nach Anspruch 7 oder 8, **dadurch gekennzeichnet, dass** der Spalt in Umfangsrichtung eine konstante Breite aufweist.

10. Vorrichtung nach einem der Ansprüche 7-9, **dadurch gekennzeichnet, dass** das Verhältnis von Spaltbreite zum Wellendurchmesser (d) zwischen 1:100 und 1:10 beträgt.

11. Vorrichtung nach einem der Ansprüche 1-10, **dadurch gekennzeichnet, dass** mit der flexiblen Welle (14) eine Spannvorrichtung zur Versteifung der Welle verbunden ist.

12. Vorrichtung nach Anspruch 11, **dadurch gekennzeichnet, dass** die flexible Welle (14) eine Verankerungsvorrichtung zum Verankern der Welle im Knochen aufweist.

## Claims

1. A device for working human and/or animal tissue, in particular an arthroscope, an endoscope or a core drill, in particular an intermedullary reamer for tubular bones, comprising
- a flexible shaft (14) with an inner channel (40),
- a working head (12) connected with the flexible shaft (14) and having openings (34) communicating with the inner channel (40), and
- a washing unit (18) with a contact surface (64) for a substantially sealed application on an object (10),
- the diameter (D) of the working head (12) being greater than the outer diameter (d) of the flexible shaft so that a washing channel (30) is formed by the outer surface of the flexible shaft (14) and a bore made in the object (10), the washing channel serving to pass a washing medium therethrough.

2. The device of claim 1, wherein the washing unit (18) is connected with a suction device to create a vacuum at the working head (12).

3. The device of claim 2, wherein the suction device is connected with the inner channel (40) of the shaft (14) so that the washing medium is sucked off through the inner channel (40) together with the removed material.

4. The device of one of claims 1-3, wherein the flexible shaft (14) has a plurality of articulately connected shaft segments (44) and a hose (50) is provided in the flexible shaft (14).

5. The device of one of claims 1-4, wherein the washing unit (18) comprises a washing head (42) in which the flexible shaft (14) is rotatably supported.

6. The device of claim 5, wherein the washing head (42) is supported for displacement in a guiding element (22) of the washing unit (18).

7. The device of one of claims 1-6, wherein the flexible shaft (14) has a plurality of shaft segments (44) with mutually engaging projections (46) and recesses (48) which engage behind each other in the longitudinal direction, leaving a gap around the shaft circumference, so that the shaft segments (44) are permanently connected in the longitudinal direction having mutual play.

8. The device of claim 7, wherein all projections (46) are of the same size and are distributed regularly over the circumference of the shaft (14).

9. The device of claim 7 or 8, wherein the gap has a constant width in the circumferential direction.

10. The device of one of claims 7-9, wherein the ratio of gap width and shaft diameter (d) is between 1:100 and 1:10.

11. The device of one of claims 1-10, wherein the flexible shaft (14) is connected to a tensioning device for stiffening the shaft.

12. The device of claim 11, wherein the flexible shaft (14) has an anchoring device for anchoring the shaft in the bone.

## Revendications

1. Dispositif pour traiter des tissus humains et/ou animaux notamment arthroscope, endoscope ou foret à noyau, en particulier foret de cavité médullaire pour des os de forme tubulaire, comprenant
un arbre flexible (14) à canal interne (40),
une tête de travail (12) reliée à l'arbre flexible (14) et présentant des orifices (34), qui sont reliés au canal interne (40), et
une unité de lavage (18) présentant une surface d'appui (64) destinée à être appliquée, de façon sensiblement étanche, sur un objet (10),
un diamètre (D) de la tête de travail (12) étant plus grand qu'un diamètre extérieur (d) de l'arbre flexible, de sorte que pour l'acheminement d'un agent de lavage, un canal de lavage (30) est formé par la surface latérale extérieure de l'arbre flexible (14) et une forure réalisée dans l'objet (10).

2. Dispositif selon la revendication 1, **caractérisé en ce que** l'unité de lavage (18) est raccordée à un dispositif d'aspiration pour la génération d'une dépression au niveau de la tête de travail (12).

3. Dispositif selon la revendication 2, **caractérisé en ce que** le dispositif d'aspiration est raccordé au canal interne (40) de l'arbre (14), afin que l'aspiration de l'agent de lavage se fasse, conjointement avec le matériau évacué, à travers le canal interne (40).

4. Dispositif selon l'une des revendications 1 à 3, **caractérisé en ce que** l'arbre flexible (14) présente plusieurs éléments d'arbre (44) reliés l'un à l'autre de façon articulée, et un tuyau souple (50) est prévu dans l'arbre flexible (14).

5. Dispositif selon l'une des revendications 1 à 4, **caractérisé en ce que** l'unité de lavage (18) présente une tête de lavage (42), dans laquelle l'arbre flexible (14) est maintenu à rotation.

6. Dispositif selon la revendication 5, **caractérisé en ce que** la tête de lavage (42) est montée à coulissement dans un organe de guidage (22) de l'unité de lavage (18).

7. Dispositif selon l'une des revendications 1 à 6, **caractérisé en ce que** l'arbre flexible (14) présente plusieurs éléments d'arbre (44) pourvus d'appendices (46) et d'évidements (48) s'engageant les uns dans les autres, qui s'accrochent l'un à l'autre en succession dans la direction longitudinale, en formant une fente courant autour de la circonférence de l'arbre, de sorte que les éléments d'arbre (44) sont inséparables en direction longitudinale et sont assemblés avec un jeu mutuel.

8. Dispositif selon la revendication 7, **caractérisé en ce que** tous les appendices (46) présentent les mêmes dimensions et sont disposés selon une répartition régulière sur la circonférence de l'arbre (14).

9. Dispositif selon la revendication 7 ou 8, **caractérisé en ce que** la fente présente une largeur constante dans la direction circonférentielle.

10. Dispositif selon l'une des revendications 7 à 9, **caractérisé en ce que** le rapport de la largeur de la fente au diamètre (d) de l'arbre se situe entre 1:100 et 1:10.

11. Dispositif selon l'une des revendications 1 à 10, **caractérisé en ce qu'**à l'arbre flexible (14) est relié un dispositif tendeur destiné à rigidifier l'arbre.

12. Dispositif selon la revendication 11, **caractérisé en ce que** l'arbre flexible (14) présente un dispositif d'ancrage destiné à ancrer l'arbre dans l'os.
